# EUROPÄISCHE PATENTSCHRIFT

(11) **EP 3 992 680 B1**
(45) Veröffentlichungstag und Bekanntmachung des Hinweises auf die Patenterteilung: **25.09.2024**
(21) Anmeldenummer: 21204917.5
(22) Anmeldetag: 27.10.2021
(51) Int. Cl.: G02B 6/06, A61B 1/07, A61B 1/00, G02B 23/24, G02B 27/00, F21V 8/00, G02B 23/26

(54) **VERFAHREN UND ANORDNUNG ZUR ADAPTIERTEN BELEUCHTUNG EINES OBJEKTS MIT LICHT**
METHOD AND APPARATUS FOR ADAPTED ILLUMINATION OF AN OBJECT WITH LIGHT
PROCÉDÉ ET AGENCEMENT D'ÉCLAIRAGE ADAPTÉ D'UN OBJET PAR UNE LUMIÈRE

(30) Priorität: 27.10.2020 DE 102020128173
(43) Veröffentlichungstag der Anmeldung: 04.05.2022
(73) Patentinhaber: Technische Universität Dresden, 01069 Dresden (DE)
(72) Erfinder: Kuschmierz, Robert, 01062 Dresden (DE); Czarske, Jürgen, 01062 Dresden (DE)
(74) Vertreter: Kailuweit & Uhlemann Patentanwälte Partnerschaft mbB

(56) Entgegenhaltungen:
- US-A1- 2018 011 309
- GIUSEPPE CALAFIORE ET AL: "Nanoimprint of a 3D structure on an optical fiber for light wavefront manipulation", NANOTECHNOLOGY, INSTITUTE OF PHYSICS PUBLISHING, BRISTOL, GB, vol. 27, no. 37, 8 August 2016 (2016-08-08), pages 375301, XP020308485, ISSN: 0957-4484, [retrieved on 20160808], DOI: 10.1088/0957-4484/27/37/375301
- GISSIBL TIMO ET AL: "Spatial beam intensity shaping using phase masks on single-mode optical fibers fabricated by femtosecond direct laser writing", OPTICA, vol. 3, no. 4, 19 April 2016 (2016-04-19), pages 448, XP055897752, DOI: 10.1364/OPTICA.3.000448

## Beschreibung

Die Erfindung betrifft ein Verfahren zur adaptierten Beleuchtung eines Objekts mit Licht mittels eines kohärenten Bündels optischer Fasern sowie eine Anordnung zur adaptierten Beleuchtung eines Objekts mit Licht.

Faseroptische Systeme in Form von flexiblen Endoskopen weisen eine hohe Zahl (bis zu mehreren 100 000) einzelner, biegsamer optischer Fasern, z. B. Glasfasern, auf. Bei einer kohärenten Anordnung der Einzelfasern eines Faserbündels kann eine optische Bildübertragung vorgenommen werden. Vorteile gegenüber starren Endoskopen, bei denen die Bildinformation über ein Stablinsensystem weitergeleitet wird, liegen insbesondere in der Kompaktheit der haarfeinen Faserbündel und den flexiblen Einsatzmöglichkeiten, auch bei stark eingeschränkter Zugänglichkeit des Beobachtungsobjekts. Ein Nachteil flexibler Endoskope liegt darin, dass der erreichbare Bildkontrast relativ gering ist. Ursachen hierfür sind insbesondere die Pixelation des Faserbündels, das Übersprechen zwischen den Einzelfasern, das zu einer Reduktion der Bildschärfe führt (Blur-Effekt), und eine Multimodigkeit der Lichtwellenführung in den Einzelfasern (Speckle-Effekt). Die Verschlechterung der Bildqualität aufgrund der beschriebenen Störungen durch das Faserbündel kann mittels einer Modulationstransferfunktion beschrieben werden. Des Weiteren erlauben flexible Faserbündel nur die Übertragung von Intensitätsprofilen und deren Abbildung mittels Linsen (Nahfeldtechnik). Aufgrund der unterschiedlichen und unbekannten optischen Weglängen der Einzelfasern ist die Phaseninformation des übertragenen Lichts nicht direkt zugänglich, so dass das Fernfeld des übertragenen Lichts unbekannt und nicht gezielt einstellbar ist.

Aus dem Stand der Technik sind Methoden der adaptiven Optik bekannt, bei denen durch den Einsatz von Wellenfrontsensoren und -modulatoren Phasenaberrationen, die durch Transmission von Licht durch optische Wellenleiter entstehen, erfasst und ausgeglichen werden können.

Beispielsweise in der DE 602 23 130 T2 sind derartige Verfahren zur Strahlsteuerung von Hochenergie-Laserstrahlen offenbart.

In Cui, M. und Yang, C. Implementation of a digital optical phase conjugation system and its application to study the robustness of turbidity suppression by phase conjugation. Optics Express 18, Nr. 4 (2010), S. 3444 wird ein Verfahren der adaptiven Optik mit offenem Regelkreis vorgestellt, das als "digitale optische Phasenkonjugation" bezeichnet wird und zur Korrektur von Phasenverzerrungen durch ein optisch trübes, insbesondere biologisches Medium geeignet ist. Dabei werden eine CCD-Kamera als Sensor und ein Flächenlichtmodulator (spatial light modulator, SLM) als Aktor eingesetzt. Das beschriebene Verfahren erfordert es, dass jedes Pixel der Kamera ein virtuelles Bild auf einem korrespondierenden Pixel des SLM erzeugt, und umgekehrt. Das Verfahren verlangt dadurch eine aufwändige Kalibrierung und einen sehr hohen Justageaufwand.

In Gu, R. Y., Mahalati, R. N. und Kahn, J. M. Design of flexible multi-mode fiberendoscope. Optics Express 23, Nr. 21 (2015), S. 26905 wird ein Endoskop mit biegsamen Multimodefasern beschrieben, dass unter Nutzung eines Teilreflektors am distalen Ende und eines SLM am proximalen Ende kalibriert werden kann. Die Kalibrierung des Endoskops und Messungen mit dem Endoskop können nicht gleichzeitig vorgenommen werden.

Die Angaben "proximal" und "distal" charakterisieren Lagebeziehungen bezüglich des Faserbündels, wobei die proximale Seite des Faserbündels die der Beleuchtungsquelle zugewandte Stirnseite ist, und die distale Seite des Faserbündels die dem Objekt zugewandte Stirnseite ist.

In der DE 10 2017 217 145 A1 wird ein Lasersystem beschrieben, welches einen Strahlhomogenisierer aufweist. Der Strahlhomogenisierer dient dabei zur Homogenisierung eines im Wesentlichen gaußförmigen Strahlprofils eines Laserlichts zu einem homogenisierten Strahlprofil. Die Homogenisierung erfolgt unter Verwendung eines optischen Elements des Strahlhomogenisierers, welches als refraktives, reflektives und/oder diffraktives optisches Element konfiguriert ist. Das offenbarte Lasersystem weist ferner eine Multimodenfaser auf und ist zu einer Modenanregung in der Multimodenfaser unter Verwendung des homogenisierten Strahlprofils konfiguriert.

Die US 2004 / 0258353 A1 offenbart ein System zur Umwandlung eines ersten elektromagnetischen Feldes in ein gewünschtes zweites elektromagnetisches Feld. Das System umfasst, beispielsweise zur Kopplung von Moden zwischen Wellenleitern oder in mikrostrukturierten Wellenleitern, einen komplexen räumlichen elektromagnetischen Feldwandler, der für den Empfang mindestens eines Teils des ersten elektromagnetischen Feldes eingerichtet ist und der für die Umwandlung des empfangenen Feldes in das gewünschte elektromagnetische Feld geeignet ist Dabei stimmt mindestens eines der ersten und zweiten Felder mit einer Mode eines mikrostrukturierten Wellenleiters überein und in Folge dessen kann ein einfallender Lichtstrahl zur Anregung einer gewünschten komplizierten Mode eines bestimmten Wellenleiters genutzt werden. Des Weiteren wird die Leistung des einfallenden Strahls exklusiv in eine bestimmte Mode eingekoppelt, wodurch ein hohes Modenunterdrückungsverhältnis anderer Moden erreicht wird.

In DE 1 942 257 C3 wird ein Bildmusteranzeigegerät offenbart. Das Gerät wiest eine Mehrzahl Mustermasken auf. Diese Mustermasken sind jeweils durch ein Lumineszenzelement und ein entsprechende ausgesendetes Lichtbündel beleuchtbar. Die durch die Lichtbündel erzeugten Lichtinformationen der Mustermasken tretenden als Abbildungslichtbündel durch ein Bündel optischer Fasern hindurch und die entsprechende Mustermaske wird auf einem Bildschirm abgebildet. Das Bildmustergerät kennzeichnet sich dadurch aus, dass jeder Mustermaske und dem gemeinsamen Bildschirm eine einzige optische Faser mit einer Querschnittsverteilung des Brechungsindex nach der Beziehung *n* = *n*₀ (1 - a *r*²) und dementsprechend abbildenden Eigenschaften derart zugeordnet ist, dass durch die optische Faser ein Bild der Mustermaske auf dem Bildschirm erzeugt wird, wobei *n*₀ als Brechungsindex im Zentrum, *n* als Brechungsindex in einem radialen Abstand *r* vom Zentrum und a als eine positive Konstanten verstanden wird.

EP 2 487 815 A1 beschreibt ein Verfahren zur Erzeugung und Kompensation von Dispersion. Ein Eingangssignal eines ersten Übertragungselements wird einer ersten Dispersion ausgesetzt, wodurch zunächst ein transformiertes Signal erzeugt wird. Ferner wird durch einen Signalgeber ein Steuersignal erzeugt und das transformierte Signal wird in Abhängigkeit des Steuersignals moduliert. Das so erzeugte modulierte Signal wird einem zweiten Übertragungselement mit einer zweiten Dispersion ausgesetzt, wobei die zweite Dispersion ein bezüglich der ersten Dispersion entgegengesetztes Vorzeichen aufweist, und erzeugt auf diesem Wege ein Ausgangssignal. Dabei wird der Dispersionswert des Ausgangssignals durch Verändern des Steuersignals angepasst.

In der US 8,585,587 B2 ist ein flexibles Endoskop beschrieben, bei dem mittels eines am proximalen Faserende angeordneten SLM die relative Phase des einfallenden Lichts verändert werden kann. Die durch die Fasern verursachte Phasenverschiebung bezüglich des einfallenden Lichts wird mittels eines Wellenfrontsensors oder Interferometers bestimmt. Dazu wird eine teilreflektive Beschichtung auf die distalen Faserenden aufgebracht. Die Bestimmung der Phasenverschiebung Δφ erfolgt dadurch nach zweimaligem Durchgang des Lichts durch das Faserbündel, gemessen wird also nicht die einfache Phasenverschiebung, sondern 2Δφ, jeweils modulo 2π. Die Messung der doppelten Phasenverschiebung hat den Nachteil, nicht eindeutig zu sein. Wird beispielsweise eine doppelte Phasenverschiebung um π gemessen, kann die einfache, durch das Faserbündel verursachte Phasenverschiebung π/2 oder - π/2 betragen. Die wahre einfache Phasenverschiebung kann nur in aufwändigen Verfahren ermittelt werden.

Um dies zu umgehen, wird in der US 2015/0015879 A1 vorgeschlagen, einen Multimode-Wellenleiter distal mittels eines virtuellen Lichtquellenbilds zu beleuchten. Betrachtet wird eine doppelt ummantelte Multimodefaser mit einem Monomodefaserkern, an deren distalem Ende ein holographisches Aufnahmematerial sowie ein punktreflexerzeugendes Objekt (wodurch eine virtuelle Lichtquelle hinter dem Objekt erzeugt wird) platziert ist. Bei Beleuchtung interferiert die durch die Monomodefaser transmittierte Welle mit dem von der virtuellen Lichtquelle ausgehenden Licht konstruktiv am Ort des Aufnahmematerials. Nachteilig ist die Position der virtuellen Lichtquelle festgelegt und kann nicht frei gewählt werden, und damit ist auch die Positionswahl für den realen Fokus stark eingeschränkt. Des Weiteren ist am distalen Faserende ein spezieller optischer Aufbau notwendig.

In der EP 3 518 017 A1 ist ein Verfahren beschrieben, mittels dessen zur Kalibrierung eines Faserbündels direkt die einfache Phasenverzerrung des Lichts, die durch Übertragung mittels des Faserbündels hervorgerufen wird, bestimmt werden kann. Dabei wird ausgenutzt, dass auf distaler Seite des Faserbündels bei Reflexion des durch eine einzelne Faser des Faserbündels geleiteten Lichts an einem halbdurchlässigen Spiegel auf der nicht dem Faserbündel zugewandten Seite des Spiegels ein virtuelles Bild der Einzelfaser entsteht, und das reflektierte Licht als von dieser annähernd punktförmigen, virtuellen Quelle ausgehend aufgefasst werden kann. Es erfolgt also eine Beleuchtung distal von der virtuellen Lichtquelle aus. Die bei der Kalibrierung ermittelte Systemfunktion wird zur Störungskorrektur bei der Beleuchtung oder Detektion eines Objekts verwendet. Dem Vorteil des direkten Zugriffs auf die eindeutige einfache Phasenverschiebung stehen bei dem Verfahren der EP 3 518 017 A1 Nachteile gegenüber, die insbesondere darauf zurückzuführen sind, dass die Störungskorrektur mittels eines Flächenlichtmodulators mit bewegbaren Einzelelementen, an dem z.B. das Licht aus dem Beleuchtungssystem vor Eintritt in das Faserbündel reflektiert wird, erfolgt. Die Einzelelemente werden so eingestellt, dass dem am Flächenlichtmodulator reflektierten Licht das Inverse der Systemfunktion aufgeprägt wird. Das Aufsetzen des Systems ist komplex, da der Flächenlichtmodulator mit einem Mikroskop auf die jeweilige Faserabgebildet werden muss. Eine Dejustage zwischen dem Flächenlichtmodulator und der Faser ist danach unbedingt zu vermeiden, wobei die Toleranzen nur im sub-µm-Bereich liegen. Nicht zuletzt fallen durch den Einsatz von Flächenlichtmodulatoren hohe Investitionskosten an.

Die US 2018/0011309 A1 beschreibt ein Verfahren und eine Vorrichtung zur Beleuchtung eines Objektes mittels eines Bündels optischer Monomode-Fasern, bei dem die Phasenverschiebung jedes Lichtstrahls Bᵢ, welcher in eine individuelle Faser eintritt, mit Hilfe einer optischen Vorrichtung moduliert wird. Die optische Vorrichtung ist beabstandet zu der Eintrittsfläche der Fasern angeordnet und enthält mindestens einen Flächenlichtmodulator sowie ein weiteres optisches System, welches die Dimension des von der optischen Vorrichtung ausgehenden Lichtstrahls an die Eintrittsfläche der Fasern anpasst

Calafiore et al. "Nanoimprint of a 3D structure on an optical fiber for light wavefrontmanipulation", NANOTECHNOLOGY 27 (2016) 375301, befasst sich mit der Fertigung von photonischen Strukturen auf der Oberfläche einer optischen Faser, wie bspw. von optischen oder teiloptischen Sensoren. Zur Herstellung der dreidimensionalen Strukturen auf der Faseroberfläche wird zunächst eine Mutterform in einem Substrat mittels lonenstrahlätzen erzeugt. Mit Hilfe eines UV-aushärtbaren Resists werden ein erster, negativer Abdruck und von diesem ein zweiter, positiver Abdruck erzeugt. Auf diesen zweiten Abdruck, der nunmehr als Gussform dient, wird die Oberfläche einer Faser, die wiederum mit einem UV-aushärtbaren Resist bedeckt ist, gepresst und das Resist mittels UV-Licht ausgehärtet. Die Strukturen nehmen jeweils nur einen geringen Teil der Oberfläche der Faser ein.

Gissibl et al. "Spatial beam intensity shaping using phase masks on single-mode optical fibers fabricated by femtosecond direct laser writing", OPTICA Vol. 3, No. 4 (2016), page 448-451, beschreibt eine Phasenmaske, die als zusätzliches, d.h. additives, Element auf der Oberfläche einer Faser aufgebracht ist. Dazu wird ein Zwei-Photonen-Laserschreibverfahren genutzt, bei dem die Oberfläche der zu beschichteten Faser in eine Photoresist-Lösung eingetaucht und das Photoresist mittels Laserbestrahlung an den gewünschten Stellen ausgehärtet wird. Auch hier ist das erzeugte Element flächenmäßig klein in Bezug auf die gesamte Querschnittsfläche der Faser, d.h. deren Oberfläche.

Aufgabe der Erfindung ist es daher, die Nachteile des Stands der Technik zu überwinden und eine Vorrichtung sowie ein Verfahren anzugeben, welche auch ohne Einsatz eines Flächenlichtmodulators die Nutzung eines Faserbündels als kohärentes Phasenarray erlauben.

Die Aufgabe wird gelöst durch ein Verfahren mit den Merkmalen des Anspruchs 1 und eine Anordnung mit den Merkmalen des Anspruchs 8. Weiterbildungen der Erfindung sind in Unteransprüchen angegeben.

Erfindungsgemäß wird die Aufgabe durch ein Verfahren zur adaptierten Beleuchtung eines Objekts mittels eines kohärenten Bündels optischer Fasern gelöst, welches zumindest folgende Verfahrensschritte aufweist:
a. Ermitteln einer die Übertragungseigenschaften zwischen dem proximalen und dem distalen Faserende des Bündels optischer Fasern widerspiegelnden Systemfunktion;
b. Formschlüssiges Anordnen einer zumindest durch das Bündel optischer Fasern hervorgerufene Übertragungsstörungen korrigierenden Phasenmaske auf mindestens einem der Faserenden des Bündels optischer Fasern, wobei die Phasenmaske so ausgestaltet ist, dass dem von einem Beleuchtungssystem emittierten und mittels des Bündels optischer Fasern zu übertragenden Licht zumindest das Inverse der Systemfunktion aufgeprägt wird;
c. Adaptierte Beleuchtung des Objekts durch proximale Beleuchtung des mit der Phasenmaske versehenen Bündels optischer Fasern mit vom Beleuchtungssystem emittierten Licht.

Ein geordnetes Faserbündel wird als "kohärent" bezeichnet, wenn die Positionsbeziehung zwischen jeweils zwei Fasern des Bündels über die gesamte Länge des Bündels erhalten bleibt. Lagebeziehungen bezüglich des Faserbündels werden im Folgenden häufig durch die Angaben "proximal" und "distal" charakterisiert, wobei die proximale Seite des Faserbündels de dem Beleuchtungssystem zugewandte Stirnseite ist, und die distale Seite des Faserbündels die dem Objekt zugewandte Stirnseite ist.

Das Bündel optischer Fasern kann insbesondere flexibel im Sinne von biegsam sein.

Die Übertragung von Licht mittels eines flexiblen Bündels optischer Fasern ist störungsbehaftet, wobei die Störungen beispielsweise als Verzerrung der Wellenfronten des Lichts bzw. als Deformation des Wellenfelds veranschaulicht werden.

Verfahrensschritt a. umfasst zumindest die Erfassung und Auswertung der die Übertragungseigenschaften zwischen dem proximalen und dem distalen Faserende des Bündels optischer Fasern widerspiegelnden Systemfunktion des Faserbündels, die durch die Transmissionsmatrix zwischen dem proximalen und dem distalen Ende des Faserbündels dargestellt werden kann und dem Fachmann auch als "Übertragungsfunktion" oder "optische Übertragungsfunktion" (optical transfer function, OTF) geläufig ist.

Die Ermittlung der Systemfunktion des Faserbündels kann prinzipiell mit jedem dafürgeeigneten Verfahren durchgeführt werden, mit dem zumindest die Phaseninformation des durch die Übertragung mittels eines Bündels optischer Fasern gestörten Lichts extrahiert werden kann. Geeignete Verfahren sind dem Fachmann bekannt.

Bevorzugt kann die Ermittlung der Systemfunktion mittels digitaler Holografie erfolgen. Detektiert und ausgewertet wird dabei die räumliche Intensitätsverteilung eines Interferenzmusters, das durch kohärente Überlagerung des durch Übertragung mittels des Faserbündels gestörten Lichts mit einer Referenzwelle entsteht. Besonders bevorzugt wird die Referenzwelle durch Strahlteilung des vom Beleuchtungssystem emittierten Lichts erzeugt. Vorteilhaft bietet die digitale Holografie die Möglichkeit, schnell und ohne aufwändige Iterationen die Phaseninformation zu bestimmen.

Ebenfalls bevorzugt kann die Ermittlung der Systemfunktion durch numerische Rekonstruktion der Phaseninformation aus der detektierten Intensitätsverteilung mittels eines Phase-retrieval-Verfahrens erfolgen. Es kann beispielsweise eine Auswertung der Transport of-Intensity Equation erfolgen, wobei dazu die Detektion der Intensitätsverteilung an mindestens zwei verschiedenen Punkten entlang des optischen Wegs vorgenommen werden muss.

Beispielsweise können zur Ermittlung der die Übertragungseigenschaften zwischen dem proximalen und dem distalen Faserende des Bündels optischer Fasern widerspiegelnden Systemfunktion zumindest folgende Schritte ausgeführt werden:
i) Proximale Beleuchtung mindestens einer frei wählbaren Einzelfaser des kohärenten Bündels optischer Fasern mit Licht;
ii) Zumindest teilweise Reflexion des Lichts an von den distalen Facetten der optischen Fasern des Bündels beabstandet angeordneten Mitteln zur teilweisen Reflexion des Lichts, so dass von dem reflektierten Licht ein mehr als eine Einzelfaser umfassender Bereich des Bündels optischer Fasern distal beleuchtet wird, wobei das reflektierte Licht dem von dem virtuellen Bild der beleuchteten Einzelfaser ausgehenden Licht entspricht;
iii) Detektion der räumlichen Intensitätsverteilung des reflektierten, durch die Übertragung mittels des distal beleuchteten Bereichs des Bündels optischer Fasern gestörten Lichts oder der räumlichen Intensitätsverteilung eines durch kohärente Überlagerung des reflektierten, durch die Übertragung mittels des distal beleuchteten Bereichs des Bündels optischer Fasern gestörten Lichts mit einer Referenzwelle erzeugten Interferenzmusters;
iv) Auswertung der detektierten Daten zumindest zur direkten Extraktion der einfachen Phasenverschiebung ΔΦ des reflektierten, durch die Übertragung mittels des distal beleuchteten Bereichs des Bündels optischer Fasern gestörten Lichts;
v) Ermittlung einer die Übertragungseigenschaften des distal beleuchteten Bereichs des Bündels optischer Fasern widerspiegelnden Systemfunktion;
vi) Wiederholung der Unterverfahrensschritte i) bis v) zur Ermittlung einer die Übertragungseigenschaften des gesamten Bündels optischer Fasern widerspiegelnden Systemfunktion.

Für das Verfahren zur Ermittlung der Systemfunktion des Faserbündels nach dem vorbeschriebenen Beispiel wird ausgenutzt, dass auf distaler Seite des Faserbündels bei Reflexion des durch eine einzelne Faser des Faserbündels geleiteten Lichts an Mitteln zur teilweisen Reflexion auf der nicht dem Faserbündel zugewandten Seite der Mittel zur teilweisen Reflexion ein virtuelles Bild der Einzelfaser entsteht, wobei das reflektierte Licht als von dieser annähernd punktförmigen, virtuellen Quelle ausgehend aufgefasst werden kann. Es erfolgt also eine Beleuchtung distal von der virtuellen Lichtquelle aus, wobei die Anzahl der distal beleuchteten Fasern des Faserbündels vom Abstand zwischen der virtuellen Lichtquelle und den distalen Facetten des Faserbündels abhängig ist. Die Anwendung der annähernd punktförmigen, virtuellen Lichtquelle weist Analogien zum aus der Astronomie bekannten Konzept des Leitsterns auf, so dass die annähernd punktförmige, virtuelle Lichtquelle im Folgenden auch als "Leitstern" bezeichnet wird.

In Unterverfahrensschritt iii) erfolgt die Detektion der räumlichen Intensitätsverteilung des reflektierten Lichts, also des durch die Übertragung mittels des von der virtuellen Lichtquelle distal beleuchteten Bereichs des Faserbündels gestörten Lichts, oder die Detektion eines Interferenzmusters, das durch kohärente Überlagerung des reflektierten, also des durch die Übertragung mittels des distal beleuchteten Bereichs des Faserbündels gestörten, Lichts mit einer Referenzwelle erzeugt wird. Dabei erlauben die detektierten Daten aufgrund der distalen Beleuchtung der Fasern durch den Leitstern die Extraktion der einfachen Phasenverschiebung ΔΦ statt der doppelten Phasenverschiebung 2·ΔΦ bei rein proximaler Beleuchtung.

Prinzipiell ist es möglich, eine einzelne Faser des Faserbündels proximal zu beleuchten und die Mittel zur teilweisen Reflexion so weit von den distalen Facetten des Faserbündels entfernt anzuordnen, dass der Leitstern in genügend weiter Entfernung zu den distalen Facetten des Faserbündels entsteht, um das gesamte Faserbündel zu beleuchten. Dann kann Unterverfahrensschritt vi) entfallen, da durch die Unterverfahrensschritte i) bis v) bereits eine die Übertragungseigenschaften des gesamten Bündels optischer Fasern widerspiegelnde Systemfunktion ermittelt wurde. Ein großer Abstand zwischen den Mitteln zur teilweisen Reflexion und den distalen Facetten des Faserbündels kann allerdings zu Abbildungsfehlern führen und die Qualität des beispielhaft beschriebenen Verfahrens zur Ermittlung der Systemfunktion negativ beeinflussen. Vorteilhaft wird das beispielhaft beschriebene Verfahren daher so angewendet, dass der Abstand zwischen den Mitteln zur teilweisen Reflexion und den distalen Facetten des Faserbündels so gewählt wird, dass nur ein Teilbereich des Faserbündels um die proximal beleuchtete Einzelfaser distal beleuchtet wird. Die Unterverfahrensschritte i) bis v) werden so oft wiederholt, bis eine die Übertragungseigenschaften des gesamten Bündels optischer Fasern widerspiegelnde Systemfunktion ermittelt wurde. Dazu werden in Unterverfahrensschritti) jeweils voneinander verschiedene Einzelfasern proximal beleuchtet, so dass verschiedene Teilbereiche des Faserbündels distal beleuchtet werden, wobei jeder Teilbereich zumindest einmal erfasst und die Systemfunktion des gesamten Bündels durch Kombination der Systemfunktionen der Teilbereiche berechnet wird. Der Abstand der Mittel zur teilweisen Reflexion von den distalen Facetten des Faserbündels kann typischerweise etwa 100 µm betragen.

Dann kann die Auswertung der detektierten Daten und die Extraktion zumindest der Phaseninformation des gestörten Lichts durch eine Messung der komplexen Lichtamplitude über digitale Holografie erfolgen. Detektiert und ausgewertet wird dabei die räumliche Intensitätsverteilung eines Interferenzmusters, das durch kohärente Überlagerung des reflektierten, gestörten Lichts mit einer Referenzwelle entsteht.

Oder die Auswertung der detektierten Daten und die Extraktion zumindest der Phaseninformation des gestörten Lichts kann durch numerische Rekonstruktion aus der detektierten Intensitätsverteilung mittels eines Phase-retrieval-Verfahrens erfolgen.

Das erfindungsgemäße Verfahren ist insbesondere geeignet für kohärente Bündel optischer Fasern, deren Systemfunktion zumindest kaum biegesensitiv ist, so dass biegeinduzierte Änderungen der Systemfunktion vernachlässigbar sind. Solche Faserbündel sind aus dem Stand der Technik bekannt, z.B. aus Tsvirkun, V., et al. Flexible lensless endoscope with conformationally invariant multi-core fiber. Optica 6, Nr. 9 (2019), S. 1185.

Verfahrensschritt b. des erfindungsgemäßen Verfahrens umfasst das formschlüssige Anordnen einer zumindest die durch die Übertragung mittels des Bündels optischer Fasern hervorgerufenen Störungen korrigierenden Phasenmaske auf mindestens einem, bevorzugt auf genau einem, der Faserenden des Bündels optischer Fasern. Die Anordnung der Phasenmaske erfolgt formschlüssig mit dem jeweiligen Faserende und demnach abstandslos zum Faserende.

Die Phasenmaske ist so ausgestaltet, dass dem von einem Beleuchtungssystem emittierten und mittels des Bündels optischer Fasern zu übertragenden Licht zumindest das Inverse der in Verfahrensschritt a. ermittelten Systemfunktion aufgeprägt wird.

Anschließend erfolgt eine adaptierte Beleuchtung des Objekts auf distaler Seite des Faserbündels durch proximale Beleuchtung des mit der Phasenmaske versehenen Bündels optischer Fasern mit Licht aus einem Beleuchtungssystem. "Adaptiert" bedeutet hier, dass das zur Beleuchtung des Objekts zur Verfügung stehende Wellenfeld nach Verlassen des Faserbündels aufgrund der Phasenmaske gegenüber einem von demselben Beleuchtungssystem emittierten Wellenfeld, das ein Faserbündel, auf dem keine Phasenmaske angeordnet ist, verlässt, verändert ist.

Dabei umfasst die Adaptierung zumindest die Korrektur der durch Übertragung des von dem Beleuchtungssystem emittierten Lichts mittels des Faserbündels hervorgerufenen Störungen.

In einer Ausführungsform des erfindungsgemäßen Verfahrens ist die Phasenmaske so ausgestaltet, dass dem von einem Beleuchtungssystem emittierten und mittels des Bündels optischer Fasern zu übertragenden Licht das Inverse der in Verfahrensschritt a. ermittelten Systemfunktion aufgeprägt wird. Damit steht nach Übertragung durch das mit der Phasenmaske versehene Bündel optischer Fasern ein weitgehend dem von dem Beleuchtungssystem emittierten entsprechendes Wellenfeld zur Beleuchtung eines Objekts zur Verfügung Es erfolgt also aufgrund der Phasenmaske eine störungskorrigierte Beleuchtung des Objekts mittels des Bündels optischer Fasern.

Vorteilhaft bietet das erfindungsgemäße Verfahren die Möglichkeit, Manipulationen der proximalen Beleuchtung vollständig in die Objektebene zu übertragen. Beispielsweise kann das Beleuchtungssystem ein adaptives System sein, wobei die Wellenfrontkrümmung und/oder Wellenfrontneigung des proximalen Wellenfelds, mit dem das Faserbündel beleuchtet wird, so manipuliert werden können, dass auf distaler Seite des Faserbündels ein durch die Manipulation des Beleuchtungssystems vorgegebenes definiertes Lichtmuster zur Beleuchtung des Objekts zur Verfügung steht. Beispielsweise kann die Beleuchtung des Faserbündels so manipuliert sein, dass distal ein Brennpunkt axial und lateral gescannt werden kann.

In einer weiteren Ausführungsform des erfindungsgemäßen Verfahrens ist die Phasenmaske so ausgestaltet, dass dem von einem Beleuchtungssystem emittierten und mittels des Bündels optischer Fasern zu übertragenden Licht das Inverse der in Verfahrensschritt a. ermittelten Systemfunktion und ein definiertes Lichtmuster aufgeprägt wird. Bei Verlassen des mit der Phasenmaske versehenen Bündels optischer Fasern steht also ein störungskorrigiertes, definiertes Lichtmuster zur Beleuchtung des Objekts zur Verfügung.

Das definierte Lichtmuster kann insbesondere mindestens einen Brennpunkt aufweisen. Vorteilhaft kann durch die Möglichkeit zur Fokussierung mittels der Phasenmaske das Verfahren für aktorische Anwendungen von Licht genutzt werden, z. B. für optische Pinzetten oder therapeutische Bestrahlungen.

In einer weiteren Ausführungsform des erfindungsgemäßen Verfahrens erfolgt in Verfahrensschritt b. das Anordnen der Phasenmaske auf mindestens einem der Faserenden des Bündels optischer Fasern formschlüssig mittels additiver Fertigung. Dabei wird mindestens eine Einzelfaser des Faserbündels gegenüber dem Ausgangszustand definiert verlängert, wobei der Grad der Verlängerung, das heißt, die axiale Abmessung der Verlängerung, zwischen voneinander verschiedenen Einzelfasern variieren kann.

Beispielsweise wird die Phasenmaske additiv mittels eines 3D-Druck-Verfahrens, z.B. mit Zwei-Photonen-Lithographie, aufgebracht.

In einer alternativen Ausführungsform des erfindungsgemäßen Verfahrens erfolgt in Verfahrensschritt b. das formschlüssige Anordnen der Phasenmaske mittels subtraktiver Fertigung, also durch Abtragen mindestens einer Einzelfaser des Faserbündels. Dabei wird mindestens eine Einzelfaser des Faserbündels gegenüber dem Ausgangszustand definiert verkürzt, wobei der Grad der Verkürzung, das heißt, die axiale Abmessung der Verkürzung, zwischen voneinander verschiedenen Einzelfasern variieren kann.

Beispielsweise wird die Phasenmaske subtraktiv mittels Laserablation an mindestens einem Faserende des Faserbündels angeordnet.

Bevorzugt erfolgt das Anordnen der Phasenmaske mittels subtraktiver Fertigung in-situ. Die Verfahrensschritte a. und b. können dann in derselben Apparatur durchgeführt werden, ohne das Faserbündel aus der für Verfahrensschritt a) verwendeten Messanordnung zu entfernen.

Ein weiterer Aspekt der Erfindung betrifft eine Anordnung zur adaptierten Beleuchtung eines Objekts mit Licht. Die Anordnung umfasst ein kohärentes Bündel optischer Fasern und eine zumindest durch das Bündel optischer Fasern hervorgerufene Übertragungsstörungen korrigierende Phasenmaske. Erfindungsgemäß ist die Phasenmaske an mindestens einem Faserende des Bündels optischer Fasern formschlüssig bezüglich des Faserendes angeordnet, wobei die Phasenmaske so ausgestaltet ist, dass dem von einem Beleuchtungssystem emittierten und mittels des Bündels optischer Fasern zu übertragenden Licht zumindest das Inverse der Systemfunktion des Bündels optischer Fasern aufgeprägt wird.

Die Phasenmaske kann so ausgestaltet sein, dass dem von einem Beleuchtungssystem emittierten und mittels des Bündels optischer Fasern zu übertragenden Licht das Inverse der Systemfunktion aufgeprägt wird, so dass nach Verlassen des Bündels optischer Fasern ein Wellenfeld zur Beleuchtung des Objekts zur Verfügung steht, dass im Wesentlichen dem vom Beleuchtungssystem emittierten entspricht. Beispielsweise kann das Beleuchtungssystem ein adaptives System sein, wobei die Wellenfrontkrümmung und/oder Wellenfrontneigung des proximalen Wellenfelds, mit dem das Faserbündel beleuchtet wird, so manipuliert werden können, dass auf distaler Seite des Faserbündels ein durch die Manipulation des Beleuchtungssystems vorgegebenes definiertes Lichtmuster zur Beleuchtung des Objekts zur Verfügung steht.

Die Phasenmaske kann aber auch so ausgestaltet sein, dass dem von einem Beleuchtungssystem emittierten und mittels des Bündels optischer Fasern zu übertragenden Licht das Inverse der in Verfahrensschritt a. ermittelten Systemfunktion und ein definiertes Lichtmuster aufgeprägt wird. Bei Verlassen der erfindungsgemäßen Anordnung steht also ein störungskorrigiertes, definiertes Lichtmuster zur Beleuchtung des Objekts zur Verfügung. Insbesondere kann das definierte Lichtmuster mindestens einen Brennpunkt aufweisen.

Das Bündel optischer Fasern kann insbesondere flexibel im Sinne von biegsam sein.

Die erfindungsgemäße Anordnung ermöglicht eine pixelfreie und beugungslimitierte Abbildung eines Objekts, wobei aufgrund der Erhaltung der Phaseninformation auch dreidimensionale Informationen des Objekts erfasst werden können.

Mit anderen Worten kann die Anordnung als kompaktes holografisches Faserbündel charakterisiert werden.

Die Anordnung hat typischerweise einen Durchmesser kleiner als 1 mm, z.B. 500 µm.

In einer Ausführungsform der erfindungsgemäßen Anordnung ist genau eine Phasenmaske an genau einem der Faserenden des kohärenten Bündels optischer Fasern angeordnet, so dass die Phasenmaske mit dem Faserende des Bündels optischer Fasern formschlüssig und demnach abstandslos abschließt. Bevorzugt ist die Phasenmaske am proximalen Faserende des Bündels optischer Fasern formschlüssig abschließend angeordnet.

In einer weiteren Ausführungsform der erfindungsgemäßen Anordnung ist durch die Phasenmaske mindestens eine Einzelfaser des Bündels optischer Fasern gegenüber dem unkorrigierten Ausgangszustand, dessen Systemfunktion zu korrigieren ist, verlängert. Die Phasenmaske ist also formschlüssig additiv an mindestens einem Faserende des Bündels optischer Fasern angeordnet. Dabei können mehr als eine Einzelfaser bis hin zu jeder Einzelfaser verlängert sein. Die axiale Abmessung der jeweiligen Verlängerung kann zwischen individuellen Einzelfasern oder zwischen Gruppen von Einzelfasern variieren.

In einer weiteren Ausführungsform der erfindungsgemäßen Anordnung ist durch die Phasenmaske mindestens eine Einzelfaser des Bündels optischer Fasern gegenüber dem unkorrigierten Ausgangszustand, dessen Systemfunktion zu korrigieren ist, verkürzt. Die Phasenmaske ist also formschlüssig subtraktiv an mindestens einem Faserende des Bündels optischer Fasern angeordnet. Dabei können mehr als eine Einzelfaser bis hin zu jeder Einzelfaser verkürzt sein. Die axiale Abmessung der Verkürzung kann zwischen individuellen Einzelfasern oder zwischen Gruppen von Einzelfasern variieren.

Ein weiterer Aspekt der Erfindung betrifft die Verwendung des erfindungsgemäßen Verfahrens oder der erfindungsgemäßen Anordnung zur 3D-Bildgebung oder zum 3D-Druck oder zur Zelldissektion und -Ablation oder zur Optogenetik. Die Erfindung kann besonders vorteilhaft in den oben genannten Anwendungen verwendet werden, da die erfindungsgemäße Anordnung ein kohärentes Phasenarray darstellt, so dass einedreidimensionale Informationsgewinnung möglich ist, das heißt, es werden nicht nur die lateralen Koordinaten, sondern auch die axiale Koordinate erfasst.

Im Rahmen dieser Beschreibung wird im Sinne der Kürze der Begriff "mindestens ein(e)" verwendet, welcher bedeuten kann: eins, genau eins, mehrere (z. B. genau zwei, oder mehr als zwei), viele (z. B. genau drei oder mehr als drei), etc. Dabei muss "mehrere" oder "viele" nicht unbedingt bedeuten, dass es mehrere oder viele identische Elemente gibt, sondern mehrere oder viele im Wesentlichen funktional gleiche Elemente.

Die Erfindung wird im Folgenden durch Ausführungsbeispiele anhand von Figuren erläutert, ohne auf diese beschränkt zu sein, da der Schutzumfang der Erfindung nur durch die anhängigen Ansprüche beschränkt ist.

Dabei zeigt die
Fig. 1 eine Messanordnung zur Ermittlung der Systemfunktion eines kohärenten Bündels optischer Fasern,
Fig. 2 eine Anordnung aus einem kohärenten Bündel optischer Fasern und einer additiv angeordneten Phasenmaske, und
Fig. 3 eine Anordnung aus einem kohärenten Bündel optischer Fasern und einer subtraktiv angeordneten Phasenmaske.

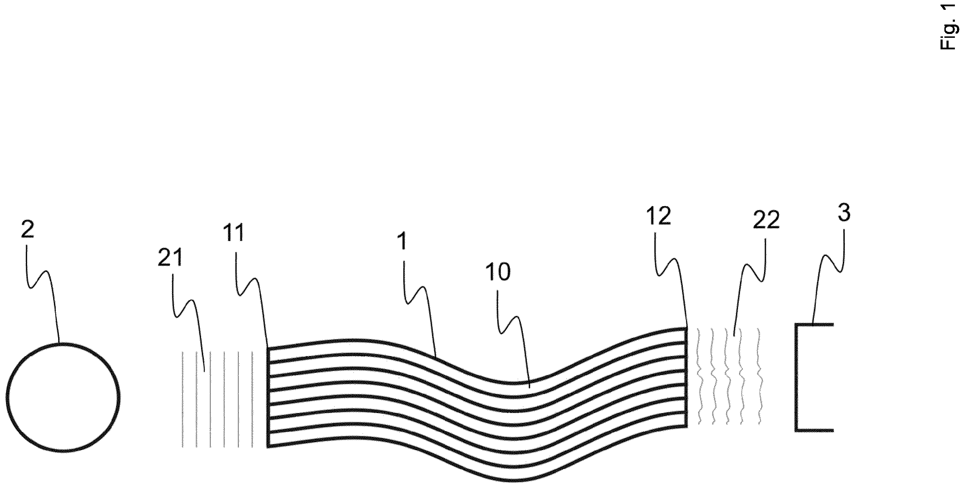

Fig. 1 zeigt ein Ausführungsbeispiel einer Messanordnung im Transmissionsmodus zur Bestimmung der Systemfunktion eines kohärenten Faserbündels 1 aus einer Vielzahl von biegsamen Einzelfasern 10. Die Messanordnung umfasst eine Beleuchtungsquelle 2, z.B. einen Laser, welche durch die ebenen Wellenfronten 21 symbolisiertes Licht emittiert. Die ebene Welle 21 tritt mit der Phase Φₑᵢₙ am proximalen Faserende 11 in das Faserbündel 1 ein und wird an das distale Faserende 12 übertragen. Anschaulich gesprochen wird dem Licht durch die Übertragung die Phase Φ_{OTF} der Systemfunktion aufgeprägt, so dass am distalen Faserende 12 eine Welle 22 mit verzerrten Wellenfronten und der Phase Φₐᵤₛ = Φₑᵢₙ + Φ_{OTF} austritt. Zur Bestimmung der Systemfunktion wird im gezeigten Beispiel die Intensitätsverteilung der verzerrten Welle 22 mit einem Detektor 3 gemessen und aus den detektierten Daten die Systemfunktion mit der Phase Φ_{OTF} numerisch rekonstruiert, z.B. mittels eines Phase-retrieval-Verfahrens.

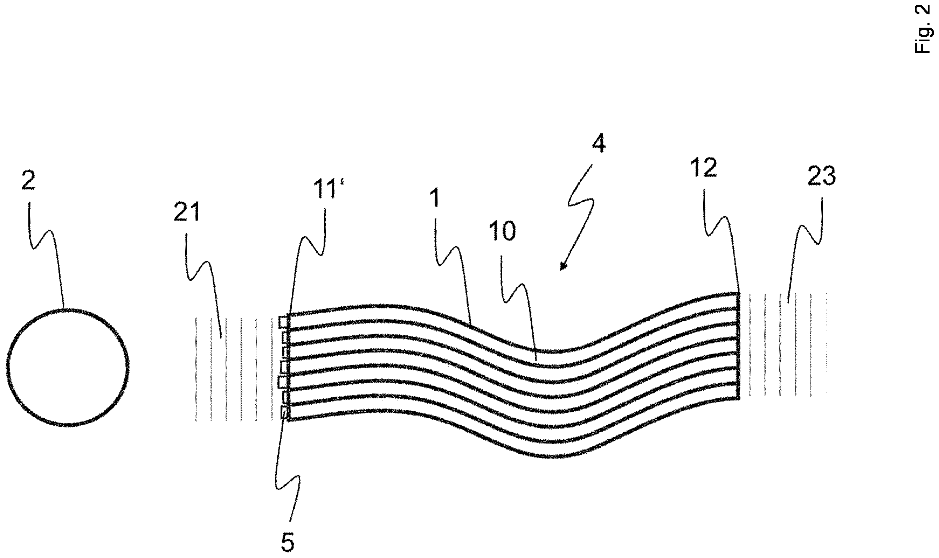

Fig. 2 zeigt eine Anordnung 4 aus einem kohärenten Faserbündel 1 mit biegsamen Einzelfasern 10 und einer Phasenmaske 5. Die Phasenmaske 5 ist mittels eines additiven Verfahrens formschlüssig auf dem proximalen Faserende 11' im unkorrigierten Ausgangszustand des Faserbündels 1, wie in der Fig. 1 gezeigt, angeordnet. Mehrere Einzelfasern 10 des Faserbündels 1 sind daher durch Aufbringen, z.B. mittels eines lithografischen Verfahrens, einer Verlängerung, z.B. aus einem optischen Polymer, gegenüber ihrer unkorrigierten Länge, wie gezeigt in Fig. 1, verlängert, wobei die axiale Abmessung der Verlängerungen von Einzelfaser zu Einzelfaser variiert.

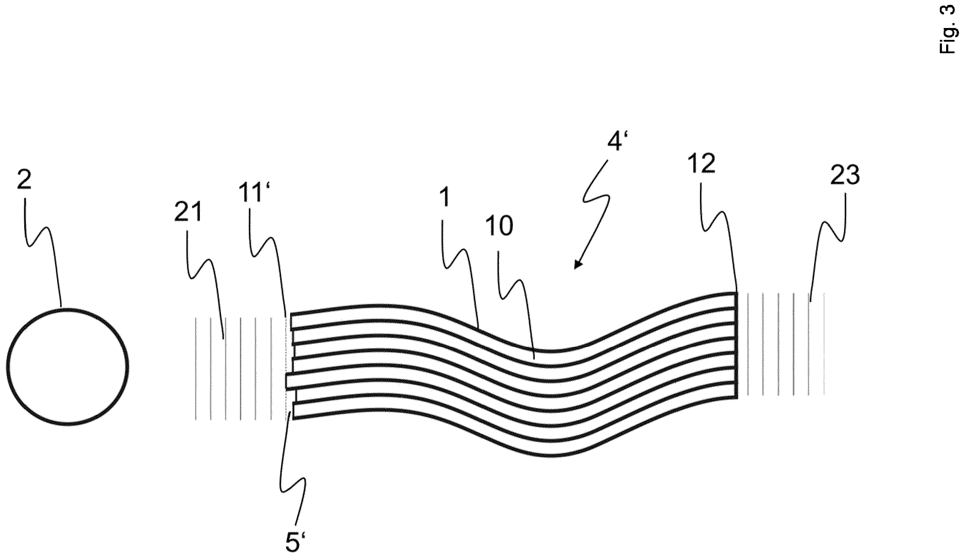

Fig. 3 zeigt eine Anordnung 4' aus einem kohärenten Faserbündel 1 mit biegsamen Einzelfasern 10 und einer Phasenmaske 5`. Die Phasenmaske 5' ist mittels eines subtraktiven Verfahrens formschlüssig auf dem proximalen Faserende 11' im unkorrigierten Ausgangszustand des Faserbündels 1, wie in der Fig. 1 gezeigt, angeordnet. Mehrere Einzelfasern 10 des Faserbündels 1 sind daher durch Abtragen ihres Materials, z.B. mittels Laserablation, gegenüber ihrer unkorrigierten Länge, wie gezeigt in Fig. 1, verkürzt, wobei die axialen Abmessungen der Verkürzungen von Einzelfaser zu Einzelfaser variieren.

Mit den Anordnungen 4 oder 4` erfolgt die Beleuchtung eines Objekts (nicht dargestellt) folgendermaßen zumindest störungskorrigiert: Die Beleuchtungsquelle 2 emittiert durch die ebenen Wellenfronten 21 symbolisiertes Licht. Die ebene Welle 21 tritt mit der Phase Φₑᵢₙ über die Phasenmaske 5 oder 5` in das Faserbündel 1 ein. Die Phasenmaske 5 oder 5` prägt der ebenen Welle 21 das Inverse der Systemfunktion in Form der Phase -Φ_{OTF} auf. Durch Übertragung an das distale Ende 12 des Faserbündels 1 wird dem Licht wiederum die Phase Φ_{OTF} aufgeprägt, so dass am distalen Faserende 12 eine ebene Welle 23 austritt, die die Phase Φₐᵤₛ = Φₑᵢₙ - Φ_{OTF} + Φ_{OTF} = Φₑᵢₙ hat. Die ebene Welle 23, mit der die Beleuchtung des Objekts erfolgt, entspricht dadurch im Wesentlichen der ebenen Welle 21, die von der Beleuchtungsquelle 2 emittiert wird. Die Phaseninformation des Lichts bleibt also bei Übertragung mit den Anordnungen 4 oder 4' erhalten.

### Bezugszeichen

- 1: Faserbündel
- 10: Einzelfaser
- 11: Proximales Faserende
- 11': Proximales Faserende des unkorrigierten Ausgangszustands
- 12: Distales Faserende
- 2: Beleuchtungsquelle
- 21: Von der Beleuchtungsquelle emittierte ebene Welle
- 22: Übertragene verzerrte Welle
- 23: Übertragene störungskorrigierte Welle
- 3: Detektor
- 4: Anordnung aus Faserbündel und additiv angeordneter Phasenmaske
- 4`: Anordnung aus Faserbündel und subtraktiv angeordneter Phasenmaske
- 5: Additive Phasenmaske
- 5': Subtraktive Phasenmaske

## Patentansprüche

1. Verfahren zur adaptierten Beleuchtung eines Objekts mit Licht mittels eines kohärenten Bündels optischer Fasern (1), aufweisend zumindest die folgenden Verfahrensschritte:
a. Ermitteln einer die Übertragungseigenschaften zwischen dem proximalen (11') und dem distalen Faserende (12) des Bündels optischer Fasern (1) widerspiegelnden Systemfunktion;
b. Anordnen einer zumindest durch das Bündel optischer Fasern (1) hervorgerufene Übertragungsstörungen korrigierenden Phasenmaske (5, 5') auf mindestens einem der Faserenden (11) des Bündels optischer Fasern (1), wobei die Phasenmaske (5, 5') so ausgestaltet ist, dass dem von einem Beleuchtungssystem (2) emittierten und mittels des Bündels optischer Fasern (1) zu übertragenden Licht (21) zumindest das Inverse der Systemfunktion aufgeprägt wird;
c. Adaptierte Beleuchtung des Objekts durch proximale Beleuchtung des mit der Phasenmaske (5, 5') versehenen Bündels optischer Fasern (1) mit Licht,
**dadurch gekennzeichnet, dass** das Anordnen der Phasenmaske (5`) auf mindestens einem der Faserenden (11) des Bündels optischer Fasern (1) formschlüssig mittels subtraktiver Fertigung durch Abtragen von mindestens einer Einzelfaser (10) oder formschlüssig mittels additiver Fertigung durch Verlängerung mindestens einer Einzelfaser (10) erfolgt.

2. Verfahren gemäß Anspruch 1, wobei
in b. die Phasenmaske (5, 5') so ausgestaltet ist, dass nach Verlassen des mit der Phasenmaske (5, 5') versehenen Bündels optischer Fasern (1) ein weitgehend dem von dem Beleuchtungssystem (2) emittierten entsprechendes Wellenfeld (23) zur Beleuchtung des Objekts zur Verfügung steht; und
in c. eine störungskorrigierte Beleuchtung des Objekts durch die proximale Beleuchtung des mit der Phasenmaske (5, 5') versehenen Bündels optischer Fasern (1) mit Licht erfolgt.

3. Verfahren gemäß Anspruch 1, wobei
die in b. angeordnete Phasenmaske (5, 5') geeignet ist, ein definiertes Lichtmuster einzustellen; und
in c. eine adaptierte Beleuchtung des Objekts mit einem definierten Lichtmuster durch proximale Beleuchtung des mit der Phasenmaske (5, 5') versehenen Bündels optischer Fasern (1) mit Licht erfolgt.

4. Verfahren nach einem der Ansprüche 1 bis 3, **dadurch gekennzeichnet, dass** das Ermitteln der Systemfunktion durch digitale Holographie oder durch ein Phase-retrieval-Verfahren erfolgt.

5. Verfahren nach einem der Ansprüche 1 bis 4, **dadurch gekennzeichnet, dass** das Anordnen der Phasenmaske (5) mittels additiver Fertigung mittels eines 3D-Druck-Verfahrens erfolgt.

6. Verfahren nach Anspruch 1, **dadurch gekennzeichnet, dass** das Anordnen der Phasenmaske (5') mittels subtraktiver Fertigung in-situ erfolgt.

7. Anordnung (4, 4') konfiguriert zur adaptierten Beleuchtung eines Objekts mit Licht, aufweisend ein kohärentes Bündel optischer Fasern (1) und eine Phasenmaske (5, 5'), **dadurch gekennzeichnet, dass** auf mindestens einem der Faserenden (11) des Bündels optischer Fasern (1) eine zumindest durch das Bündel optischer Fasern (1) hervorgerufene Übertragungsstörungen korrigierende Phasenmaske (5, 5') formschlüssig bezüglich des Faserendes (11) angeordnet ist, wobei die Phasenmaske (5, 5') so ausgestaltet ist,
i dass dem von einem Beleuchtungssystem emittierten und mittels des Bündels optischer Fasern zu übertragenden Licht zumindest das Inverse der Systemfunktion des Bündels optischer Fasern aufgeprägt wird sowie
ii dass durch die Phasenmaske (5) mindestens eine Einzelfaser (10) des Bündels optischer Fasern (1) gegenüber dem unkorrigierten Ausgangszustand verlängert oder verkürzt ist.

8. Anordnung nach Anspruch 7, **dadurch gekennzeichnet, dass** genau eine Phasenmaske (5, 5') so auf einem der Faserenden (11) des Bündels optischer Fasern (1) angeordnet ist, dass sie mit dem Faserende (11) des Bündels optischer Fasern (1) formschlüssig abschließt.

9. Verwendung eines Verfahrens nach einem der Ansprüche 1 bis 6 oder einer Anordnung (4, 4') nach einem der Ansprüche 7 oder 8 zur 3D-Bildgebung oder zum 3D-Druck oder zur Zelldissektion und -Ablation oder zur Optogenetik.

## Claims

1. A method for the adapted illumination of an object with light by means of a coherent bundle of optical fibers (1), comprising at least the following method steps:
a. determining a system function reflecting the transmission characteristics between the proximal fiber end (11') and the distal fiber end (12) of the bundle of optical fibers (1);
b. arranging a phase mask (5, 5'), which corrects transmission disturbances caused at least by the bundle of optical fibers (1), on at least one of the fiber ends (11) of the bundle of optical fibers (1), the phase mask (5, 5') being designed in such a way that at least the inverse of the system function is impressed on the light (21) emitted by an illumination system (2) and to be transmitted by means of the bundle of optical fibers (1);
c. adapted illumination of the object by proximal illumination of the bundle of optical fibers (1) provided with the phase mask (5, 5') with light,
**characterized in that** the phase mask (5') is form-fitting arranged on at least one of the fibre ends (11) of the bundle of optical fibres (1) by means of subtractive manufacturing by removing at least one individual fibre (10) and/or by means of additive manufacturing by extending at least one individual fibre (10).

2. The method according to claim 1, wherein
in b. the phase mask (5, 5') is designed such that, after leaving the bundle of optical fibers (1) provided with the phase mask (5, 5'), a wave field (23) largely corresponding to that emitted by the illumination system (2) is available for illuminating the object; and
in c. interference-corrected illumination of the object is provided by the proximal illumination of the bundle of optical fibers (1) provided with the phase mask (5, 5') with light.

3. The method according to claim 1, wherein
the phase mask (5, 5') arranged in b. is suitable for setting a defined light pattern; and
in c. an adapted illumination of the object with a defined light pattern is provided by proximal illumination of the bundle of optical fibers (1) provided with the phase mask (5, 5') with light.

4. The method according to one of claims 1 to 3, **characterized in that** the determination of the system function is carried out by digital holography or by a phase-retrieval method.

5. The method according to one of claims 1 to 4, **characterized in that** the phase mask (5) is arranged by means of additive manufacturing using a 3D printing process.

6. The method according to claim 1, **characterized in that** arranging the phase mask (5') is carried out in-situ by means of subtractive manufacturing.

7. An arrangement (4, 4') configured for the adapted illumination of an object with light, having a coherent bundle of optical fibres (1) and a phase mask (5, 5'), **characterized in that** a phase mask (5, 5') which corrects transmission disturbances caused at least by the bundle of optical fibres (1) is arranged on at least one of the fibre ends (11) of the bundle of optical fibres (1) in a form-fitting manner with respect to the fibre end (11), the phase mask (5, 5') being designed such that
i. at least the inverse of the system function of the bundle of optical fibers is impressed on the light emitted by an illumination system and to be transmitted by means of the bundle of optical fibers, and
ii. at least one individual fiber (10) of the bundle of optical fibers (1) is lengthened or shortened by the phase mask (5) compared to the uncorrected initial state.

8. The arrangement according to claim 7, **characterized in that** exactly one phase mask (5, 5') is arranged on one of the fibre ends (11) of the bundle of optical fibres (1) in such a way that it terminates in a form-fitting manner with the fibre end (11) of the bundle of optical fibres (1).

9. Use of a method according to one of claims 1 to 6 or of an arrangement (4, 4') according to one of claims 7 or 8 for 3D imaging or for 3D printing or for cell dissection and cell ablation or for optogenetics.

## Revendications

1. Procédé permettant l'éclairage adapté d'un objet avec de la lumière au moyen d'un faisceau de fibres optiques (1) cohérent, présentant au moins les étapes de procédé suivantes :
a. détermination d'une fonction système reflétant les caractéristiques de transmission entre l'extrémité de fibre proximale (11') et l'extrémité de fibre distale (12) du faisceau de fibres optiques (1) ;
b. agencement d'un masque de phase (5, 5') corrigeant au moins des interférences de transmission provoquées par le faisceau de fibres optiques (1) sur au moins l'une des extrémités de fibre (11) du faisceau de fibres optiques (1), dans lequel le masque de phase (5, 5') est configuré de sorte qu'au moins l'inverse de la fonction système est appliquée à la lumière (21) destinée à être émise par un système d'éclairage (2) et devant être transmise au moyen du faisceau de fibres optiques (1) ;
c. éclairage adapté de l'objet par un éclairage proximal du faisceau de fibres optiques (1) pourvu du masque de phase (5, 5') avec de la lumière,
**caractérisé en ce que** l'agencement du masque de phase (5') sur au moins l'une des extrémités de fibre (11) du faisceau de fibres optiques (1) est effectué par complémentarité de forme au moyen d'une fabrication soustractive en ablatant au moins une monofibre (10) ou par complémentarité de forme au moyen d'une fabrication additive en prolongeant au moins une monofibre (10).

2. Procédé selon la revendication 1, dans lequel
en b., le masque de phase (5, 5') est configuré de sorte que, après avoir quitté le faisceau de fibres optiques (1) pourvu du masque de phase (5, 5'), un champ d'ondes (23) correspondant dans une large mesure à celui émis par le système d'éclairage (2) est disponible pour l'éclairage de l'objet ; et
en c., un éclairage à correction d'interférences de l'objet est effectué par l'éclairage proximal du faisceau de fibres optiques (1) pourvu du masque de phase (5, 5') avec de la lumière.

3. Procédé selon la revendication 1, dans lequel
le masque de phase (5, 5') agencé en b. est apte à régler un modèle de lumière défini ; et
en c., un éclairage adapté de l'objet est effectué par un modèle de lumière défini par l'éclairage proximal du faisceau de fibres optiques (1) pourvu du masque de phase (5, 5') avec de la lumière.

4. Procédé selon l'une des revendications 1 à 3, **caractérisé en ce que** la détermination de la fonction système est effectuée par holographie numérique ou par un procédé de récupération de phase.

5. Procédé selon l'une des revendications 1 à 4, **caractérisé en ce que** l'agencement du masque de phase (5) est effectué au moyen d'une fabrication additive au moyen d'un procédé d'impression 3D.

6. Procédé selon la revendication 1, **caractérisé en ce que** l'agencement du masque de phase (5') est effectué in situ au moyen d'une fabrication soustractive.

7. Agencement (4, 4') configuré pour l'éclairage adapté d'un objet, présentant un faisceau de fibres optiques (1) cohérent et un masque de phase (5, 5') avec de la lumière, **caractérisé en ce qu'un** masque de phase (5, 5') corrigeant au moins des interférences de transmission provoquées par le faisceau de fibres optiques (1) est agencé sur au moins l'une des extrémités de fibre (11) du faisceau de fibres optiques (1) par complémentarité de forme par rapport à l'extrémité de fibre (11), dans lequel le masque de phase (5, 5') est configuré de sorte
i qu'au moins l'inverse de la fonction système du faisceau de fibres optiques est appliquée à la lumière émise par un système d'éclairage et devant être transmise au moyen du faisceau de fibres optiques, ainsi que
ii qu'au moins une monofibre (10) du faisceau de fibres optiques (1) est prolongée ou raccourcie par le masque de phase (5) par rapport à l'état initial non corrigé.

8. Agencement selon la revendication 7, **caractérisé en ce qu'**exactement un masque de phase (5, 5') est agencé sur l'une des extrémités de fibre (11) du faisceau de fibres optiques (1) de sorte qu'il se termine par complémentarité de forme avec l'extrémité de fibre (11) du faisceau de fibres optiques (1).

9. Utilisation d'un procédé selon l'une des revendications 1 à 6 ou d'un agencement (4, 4') selon l'une des revendications 7 ou 8 pour l'imagerie 3D ou pour l'impression 3D ou pour la dissection et l'ablation de cellules ou pour l'optogénétique.
